# FASCICULE DE BREVET EUROPEEN

(11) **EP 3 405 207 B1**
(45) Date de publication et mention de la délivrance du brevet: **07.10.2020**
(21) Numéro de dépôt: 17706271.8
(22) Date de dépôt: 16.01.2017
(51) Int. Cl.: A61K 36/67, A23L 33/105, A23L 2/52, A61K 9/48, A61K 9/20, A61K 9/16, A61K 9/14, A61K 9/00, A61P 1/14, A61P 1/16

(54) **COMPOSITION A VISÉE DÉTOXIFIANTE POUR ADMINISTRATION PAR VOIE ORALE ET SON PROCÉDÉ DE PRÉPARATION**
ENTGIFTUNGSZUSAMMENSETZUNG ZUR ORALEN VERABREICHUNG UND VERFAHREN ZUR HERSTELLUNG DAVON
DETOXIFYING COMPOSITION FOR ORAL ADMINISTRATION AND METHOD FOR PREPARING SAME

(30) Priorité: 21.01.2016 FR 1650470
(43) Date de publication de la demande: 28.11.2018
(73) Titulaire: Hay, Ly Eang, Phnom Penh (KH)
(72) Inventeur: Hay, Ly Eang, Phnom Penh (KH)
(74) Mandataire: Ipsilon
(86) Numéro de dépôt international: PCT/FR2017/050088
(87) Numéro de publication internationale: WO 2017/125669

(56) Documents cités:
- WO-A2-2012/125772
- MEGHWAL MURLIDHAR ET AL: "Piper nigrum and Piperine: An Update", PHYTOTHERAPY RESEARCH, vol. 27, no. 8, août 2013 (2013-08), pages 1121-1130, XP055303768,
- TRIPATHI A K ET AL: "Bioactivities of 3-methyl-5-decanoylpyridine from Piper retrofractum (Piperaceae) towards four stored product beetles", MEDICINAL & AROMATIC PLANTS ABSTRACTS, SCIENTIFIC PUBLISHERS, SCIENTIFIC PUBLISHERS, NEW DELHI - INDIA, vol. 25, no. 4, 1 août 2003 (2003-08-01), XP018014742, ISSN: 0250-4367
- PARMAR V S ET AL: "Polyphenols and alkaloids from piper species", PHYTOCHEMISTRY, PERGAMON PRESS, GB, vol. 49, no. 4, 27 octobre 1998 (1998-10-27), pages 1069-1078, XP004290159, ISSN: 0031-9422, DOI: 10.1016/S0031-9422(98)00208-8

## Description

### DOMAINE DE L'INVENTION

La présente invention se rapporte au domaine de la détoxification et de l'élimination des déchets de l'organisme.

Plus particulièrement, la présente invention concerne une composition à visée détoxifiante renfermant un actif issu de végétaux.

La présente invention se rapporte également à la préparation de ladite composition et son utilisation pour favoriser l'élimination par voie hépatique des déchets de l'organisme.

### ETAT DE LA TECHNIQUE

La fabrication, l'utilisation, et la libération des substances xénobiotiques, y compris des produits chimiques synthétiques et des métaux ont augmenté de façon exponentielle et se retrouvent dans l'environnement. Par conséquent, l'exposition à ces substances toxiques persistantes et bioaccumulables par les humains s'est accrue. Cette exposition aux toxiques a été associée à des effets néfastes sur la santé, parmi lesquels une perturbation endocrinienne, des effets neurologiques, des effets sur la reproduction ainsi que des cancers et des maladies cardiovasculaires. Parmi ces produits chimiques associés à des maladies et des troubles comprennent les pesticides organochlorés, les phtalates, le bisphénol A, et les poly bromo diphényl éthers.

Le régime alimentaire humain renferme une grande variété de molécules naturelles cancérogènes et anticancérogènes. Un moyen d'identifier des agents chémopréventifs potentiels parmi les constituants alimentaires est de suivre les variations des niveaux d'enzymes du système de biotransformation pouvant détoxifier des produits chimiques cancérogènes. En effet, le métabolisme des xénobiotiques et composés endobiotiques implique des systèmes enzymatiques conduisant à des produits rendus inactifs permettant leur élimination de l'organisme.

Le terme "détoxification" est devenu courant dans notre société et est généralement associé à la perte de poids, la récupération à une addiction, ou à une panacée pour des désagréments nombreux et non précis. Dans la présente demande, les termes « détoxification » ou « détoxifiant » suivent la définition scientifique qui est beaucoup plus précise et concerne le processus d'élimination des poisons ou toxines ou de leurs effets d'une zone ou d'un individu, ainsi que la conversion de produits toxiques en substrats non toxiques ou toutes les réactions, catalysées par enzymes ou non, permettant de consommer des métabolites toxiques sans produire de dommage.

Cette conversion peut notamment être réalisée par le foie, selon deux voies bien établies pour la modification chimique des composés dits « toxiques ». Ces deux voies hépatiques sont communément appelées Phase I et Phase II. La Phase I implique l'utilisation d'un groupe microsomal d'isoenzymes P450. Ces oxydases à fonction mixte impliquent généralement des réactions d'oxydation, de réduction, d'hydrolyse, d'hydratation et d'isomérisation. La toxine est rendue plus soluble dans l'eau par ces réactions et donc plus facilement apte à être excrétée (i.e. moins « toxique » pour l'organisme). Les enzymes de la voie de Phase I sont connus pour métaboliser de nombreux médicaments (par exemple le phénobarbital), des hydrocarbures de la pétrochimie, ainsi que des hormones stéroïdes, telles que les estrogènes et la testostérone. La voie de Phase II conjugue les métabolites de la Phase I, ou des toxines d'origine, à des composés plus hydrophiles. Les substrats des réactions de Phase II comprennent le glutathion, le glucuronide, la glycine, et les groupements sulfure, acétyle, et méthyle. Si ces produits biotransformés ont un poids moléculaire faible, ils sont éliminés par les urines. Si les produits finaux sont d'un poids moléculaire élevé, ils sont éliminés par la bile.

De manière traditionnelle, ont été relevées les propriétés dites détoxifiantes hépatique et rénale de végétaux tels que le chardon Marie (*Silybum marianum*), la reine des prés (*Filipendula ulmaria*), le radis noir (*Raphanus sativus* var. *niger*), l'artichaut (*Cynara scolymus*), le pissenlit (*Taraxacum officinale*)*,* le boldo (*Peumus boldus*) ou de molécules telles que la glycine ou la bétaïne.

Des données ont montré des propriétés détoxifiantes du poivre et son action sur les enzymes de détoxification (A. Singh, A.R. Rao, Evaluation of the modulatory influence of black pepper (Piper nigrum, L.) on the hepatic détoxification system cancer letters, 72, pp 5-9, 1993 ; K. Singletary, Black Pepper-Overview of health benefits Nutrition today, Vol. 45, 1, pp 43-47, Janv/Fev 2010 ; I.M. Scott et al., A review of Piper spp (Piperaceae) phytochemistry, insectidical activity and mode of action, Phytochem Rev., 7, pp 65-75, 2008). Le poivre noir de *Piper nigrum* L. est l'épice la plus couramment utilisée dans le monde, et ses extraits ont été utilisés en médecine traditionnelle dans de nombreuses cultures. Le poivrier est une liane à tige ligneuse volubile fixée sur son support par ses rameaux latéraux. Les feuilles à limbe ovale aigu et parcouru par 3-4 nervures presque parallèles sont alternes, les fleurs apérianthées et sessiles sont groupées en épis pendants de 20-30 unités. Le fruit est une baie de 4-8 mm de diamètre, passant du vert au rouge au cours de la maturation. On distingue classiquement :
- Le poivre vert, qui est la baie entière fraiche cueillie verte ;
- Le poivre blanc, qui est le fruit récolté à pleine maturité. Après plusieurs jours d'immersion dans l'eau les fruits sont débarrassés du péricarpe et de la partie externe du mésocarpe puis séchés ;
- Le poivre noir, pour lequel les épis sont récoltés dès que les premières baies virent au rouge. Après séchage, les fruits sont séparés des rafles.

*Piper nigrum* est utilisé pour la production à la fois du poivre noir (à partir du fruit non affiné) et du poivre blanc (à partir de la baie mature décortiquée). Dans divers écrits anciens de diverses cultures, Inde, Chine, le poivre est utilisé, outre comme épice, à des fins médicinales pour le traitement de troubles gastro-intestinaux ou l'épilepsie. Les constituants du poivre noir comprennent des fibres, des huiles essentielles, de la pipérine, de l'eugénol, de la lipase et des minéraux.

La 1-pipéroylpipéridine, aussi nommée pipérine, est le principal composant bioactif présent à la fois dans le poivre noir et le poivre blanc. Les données scientifiques (Singletary, 2010) mettent en évidence les effets sur la santé du poivre noir, en particulier dans le renforcement de la fonction digestive. Il a également été montré, par exemple, chez le rat et la souris, que le poivre noir et la pipérine peuvent stimuler les enzymes digestives, modifier les sécrétions de l'estomac, modifier le temps de transit gastro-intestinal de la nourriture, et inhiber la diarrhée. Le poivre noir a également un effet important sur le système enzymatique métabolisant les médicaments et les produits phytochimiques. En effet, d'une part il a été montré (Scott, 2008) que la pipérine inhibe les activités d'enzymes de détoxification : arylhydrocarbon hydroxylase (AHH) et 7-éthoxycoumarin dééthylase (7ECDE); méthoxycoumarin déméthylase (MOCD); ainsi que le principal enzyme de métabolisation des drogues Cyp3a4. D'autre part, la pipérine induit les enzymes de Phase I (cytochrome b5, cytochrome P450) et de Phase II (glutathion S-transférase GST, acid-soluble sulfhydryl -SH, malondialdéhyde MDA) PSMO (Polysubstrate mono oxygénase). L'induction de la transcription des gènes des enzymes de Phase I et II a été confirmée. Un extrait éthanolique de poivre régule à la hausse les cytochromes P450 (Cyp6a8, Cyp9b2, Cyp12d1, Cyp6d4, Cyp6d5, et Cyp6w1) avec les glutathion-S-transférase S1 et glutathion-S-transférase E7.

Ces données de l'art antérieur démontrent donc le lien entre pipérine et/ou *Piper nigrum* graine/fruit et la détoxification. Le document de MEGHWAL MURLIDHAR et al.: "Piper nigrum and Piperine: An Update",Phytotherapy Research, vol. 27, no. 8, août 2013 pages 1121-1130 divulgue que les produits issus des grains de poivre sont connus pour leur activité détoxifiante.

Or le présent Demandeur a constaté, dans sa recherche d'utilisation et de valorisation d'autres parties de la plante de poivrier, que, de manière surprenante, les tiges d'inflorescence de poivrier, malgré des teneurs plus faibles en pipérine, possèdent une action de détoxification, en particulier de détoxification hépatique.

### DESCRIPTION DE L'INVENTION

La présente invention concerne donc une composition à visée détoxifiante pour son utilisation par administration par voie orale en tant que détoxifianten vue de favoriser l'élimination par voie hépatique des déchets de l'organisme, caractérisée en ce qu'elle comprend des tiges d'inflorescence de poivrier séchées, éventuellement broyées, et éventuellement tamisées et/ou un extrait, aqueux ou sec, de tiges d'inflorescence de poivrier.

Les tiges d'inflorescence de poivrier sont, de préférence choisies parmi les tiges d'inflorescence de poivrier *Piper nigrum* ou *Piper longum,* plus particulièrement les tiges d'inflorescence de poivrier de la région de Kampot, au Cambodge. Ceci permet de valoriser l'inflorescence de poivre, particulièrement celle de Kampot (Cambodge) portant le label IG Kampot distingué par le Comité Cambodgien des Indications Géographiques et certifié par l'institution internationale (Ecocert). En effet, depuis plusieurs siècles, les spécialistes de poivre du monde entier apprécient le poivre (c'est-à-dire les baies) de Kampot pour sa saveur unique et ses effets bénéfiques pour la santé. Jusqu'à présent, la tige de l'inflorescence du poivrier n'a pas été décrite pour avoir un effet particulier.

L'extrait est avantageusement un extrait desdites tiges d'inflorescence avec un solvant polaire, tel que l'eau, ou un mélange hydro-alcoolique. Ainsi il est très facile d'obtenir une composition détoxifiante buvable par simple infusion, décoction ou macération dans l'eau de tiges d'inflorescence de poivrier.

Par infusion, on entend, ici, verser de l'eau (solvant polaire) se trouvant de préférence à température d'ébullition sur la tige d'inflorescence de poivrier (par exemple 2,5 g de matières sèches /100 ml d'eau), puis laisser infuser pendant une période allant de une minute à 2 heures. Par décoction on entend la même opération, le liquide étant maintenu à ébullition. Quant à la macération, elle s'effectue avec un solvant froid. L'infusion est préférée.

Ainsi la composition pour son utilistion selon l'invention peut se présenter sous forme buvable, telle que sous la forme d'une infusion, d'une décoction, d'une macération, d'une boisson aromatisée ou toute autre forme buvable.

Les tests expérimentaux effectués par le demandeur indiquent que par exemple l'infusion de cette partie d'inflorescence séchée donne une boisson ayant un arôme spécifique agréable et stimulant. Sa consommation aide la digestion en facilitant la salivation et augmente aussi les enzymes digestives, participe à l'élimination des toxines de l'organisme. Cette boisson a aussi des propriétés relaxantes et tonifiantes.

La composition pour son utilisation selon l'invention renferme avantageusement au moins 1 % en poids, de préférence de 2 % à 15 % en poids, de préférence encore de 3 % à 10 %, de polyphénols totaux issus de la tige d'inflorescence de poivrier, ce qui lui confère des capacités à la fois anti-oxydante et détoxifiante. Par exemple les essais réalisés indiquent que la tige d'inflorescence du poivrier (*Piper nigrum*) sous forme de poudre comprend environ 3 % en poids de polyphénols.

La présente invention concerne également un complément alimentaire ou un aliment fortifié ou un produit diététique comprenant la composition décrite ci-dessus dans des proportions pondérales comprises entre 0,1 % et 85 %, de préférence entre 3 % et 65 %, de préférence encore entre 20 % et 60 % pour son utilisation par administration par voie orale en tant que détoxifiant en vue de favoriser l'élimination par voie hépatique des déchets de l'organisme.

Par complément alimentaire, on entend, ici, une denrée alimentaire dont le but est de compléter un régime normal et qui constitue une source concentrée de nutriments ou d'autres substances ayant un effet nutritionnel ou physiologique, seul ou combiné, commercialisé sous forme de doses. Par dose, on entend toutes les formes classiques : gélules, pastilles, comprimés, ampoules, ou encore sachets.

Lorsque les compléments alimentaires se présentent sous forme de capsule molle ou de gélule, l'enveloppe de ces capsules molles ou de ces gélules peut contenir notamment de la gélatine animale telle que la gélatine de poisson, de la glycérine, ou un matériau d'origine végétale tel qu'un dérivé de cellulose ou d'amidon, ou une protéine végétale.

Lorsque les compléments alimentaires se présentent sous forme de gélule, de comprimé, ou de granule, le mélange d'actifs peut être fixé sur un support pulvérulent tel que la silice, la cellulose ou la maltodextrine.

Par aliments fortifiés on entend ici des produits, tels que des yaourts ou des boissons, auxquels on a rajouté des substances nutritives, dans lesquels on a dissous la poudre ou l'extrait de tige d'inflorescence de poivrier selon l'invention.

Sont considérées comme des produits diététiques destinés à une alimentation particulière les denrées alimentaires qui, du fait de leur composition particulière ou du procédé particulier de leur fabrication, se distinguent nettement des denrées alimentaires de consommation courante, conviennent à un objectif nutritionnel indiqué et sont commercialisées en indiquant qu'elles répondent à cet objectif.

Le complément alimentaire ou aliment fortifié ou produit diététique pour une utilisation selon l'invention peut comprendre en outre au moins un actif choisi parmi : les vitamines, telles que les vitamines B6 ou C ; les caroténoïdes, tels que le bêta-carotène ou la lutéine ; les oligoéléments minéraux, tels que le magnésium, le calcium ou le fer ; ou des extraits de romarin, de chardon marie, de radis noir, de la reine des prés, de l'ortie, de la prêle, des feuilles d'orthosiphon, de la citronnelle, du gingembre, du jasmin; des polyphénols et/ou des anthocyanes additionnels.

D'autres agents acceptables sur les plans pharmaceutique et/ou alimentaire peuvent être rajoutés à l'infusion détoxifiante, tels que des agents de charge, des fluidisants, des extraits naturels, d'autres vitamines, des minéraux, des oligoéléments, des acides aminés, des acides gras, des anti-agglomérants, des colorants, des acidifiants, des épaississants, des conservateurs, des édulcorants, des adjuvants, des colorants, des arômes.

Les charges sont avantageusement choisies parmi la cellulose microcristalline ou la maltodextrine de pomme de terre ou de maïs.

L'épaississant utilisé est, de préférence, choisi parmi : l'amidon de pomme de terre, l'hydroxy propyl méthyl cellulose, la pectine de citrus, la gomme de guar, de caroube, l'agar-agar, le konjac, les huiles hydrogénées, la cire d'abeille ou un mélange de ceux-ci.

Les fluidisants peuvent être du silicate de magnésium, du stéarate de magnésium ou de la silice colloïdale.

Les anti-agglomérants sont ceux utilisés usuellement dans l'industrie alimentaire, tels que le stéarate de magnésium ou la silice colloïdale.

Les autres vitamines peuvent être choisies parmi, entre autres, les vitamines B1, B2, B3, B5, B8, B9, B12, D ou E.

En tant qu'acidifiant, de l'acide citrique peut aussi être utilisé dans la composition selon la présente invention.

Les stabilisants employés dans la fabrication du complément alimentaire ou aliment fortifié ou produit diététique pour une utilisation selon la présente invention sont ceux usuellement utilisés dans l'industrie alimentaire, par exemple les dérivés de sucres et/ou de céréales, tels que le sorbitol.

Les édulcorants pouvant être employés sont, entre autres, l'inuline, le xylitol, l'aspartame, le sirop de glucose, le sirop de fructo-oligosaccharide, le maltitol en poudre ou en sirop, l'acésulfame de potassium, le fructo-oligosaccharide, le sucralose, la néohespéridine, les stéviosides et/ou le cyclamate de sodium.

Des acides gras peuvent également être ajoutés à la composition, ils peuvent être choisis parmi les acides gras de type oméga 3 et/ou oméga 6, ou les galactolipides. Des acides aminés, tels que le tryptophane, la leucine, l'arginine, la glycine et/ou la méthionine peuvent être ajoutés.

La présence d'arômes n'est plus nécessaire, la tige d'inflorescence de poivrier de Kampot possédant des propriétés organoleptiques conférant à la composition des arômes particuliers.

Les conservateurs sont utiles afin que composition détoxifiante ou ledit complément alimentaire ou aliment fortifié ou produit diététique se conserve dans le temps. Les conservateurs utilisés peuvent être par exemple le sorbate de potassium, les parabènes, le benzoate de sodium, l'acide citrique, l'acide benzoïque, le romarin ou le palmitate d'ascorbyle (antioxydant).

Tous ces composés ne sont nullement limitatifs, des agents acceptables sur les plans pharmaceutique et/ou alimentaire pouvant être ajoutés à la composition détoxifiante ou au dit complément alimentaire ou aliment fortifié ou produit diététique selon la présente invention. Le complément alimentaire, le produit diététique ou l'aliment fortifié peut se présenter sous forme de comprimés nus ou enrobés, de comprimés multicouches, de comprimés effervescents, de dragées, de gélules, de capsules, de poudre, de granules, de sticks, de pastilles, de capsules molles, d'une solution ou d'une suspension aqueuse dans une ampoule en verre, en stick ou en boisson, en flacon compte-gouttes, ou en spray.

Avantageusement, le complément alimentaire, le produit diététique ou l'aliment fortifié est administré à l'Homme, une à quatre fois par jour, sous la forme de dose comprenant de 5 mg à 4 g, de préférence de 10 mg à 2 g de ladite composition détoxifiante selon l'invention et ce, pendant une période pouvant aller de 1 jour à 3 mois.

La présente invention concerne également le procédé de préparation de la composition détoxifiante telle que décrite ci-dessus : ce procédé comprend au moins l'une des étapes i) ou ii) suivantes de :
i) récolte, séchage, éventuel broyage, et éventuel tamisage, de tiges d'inflorescence de poivrier ;
ii) extraction de tiges d'inflorescence de poivrier fraîches et/ou séchées au moyen d'un solvant polaire, tel que l'eau, l'alcool ou un mélange hydro-alcoolique, puis filtration de l'extrait, en option concentration de l'extrait, éventuellement jusqu'à siccité.

L'extraction peut être effectuée selon différents protocoles :
- la décoction comprend la mise à ébullition de la tige d'inflorescence dans un solvant polaire et le maintien de cette ébullition pendant une durée particulière, notamment de 1 minute à 2 heures, en particulier environ 20 minutes, puis éventuellement un retour à température ambiante.
- la macération consiste à laisser tremper à froid la tige d'inflorescence de poivrier dans le solvant polaire pendant une durée particulière, afin d'en extraire les composés solubles. En particulier, l'étape de macération a une durée allant de 1 heure à 15 jours, et de manière préférée de 1 jour. Tout particulièrement, elle se fait à température ambiante, soit de 10 à 25°C.
- l'infusion, comme décrit plus haut, consiste à verser le solvant polaire se trouvant de préférence à température d'ébullition sur la tige d'inflorescence de poivrier, puis à laisser infuser pendant une période allant de une minute à 2 heures.
- la digestion comprend le chauffage de la tige d'inflorescence de poivrier dans le solvant polaire à une température inférieure à la température d'ébullition, par exemple dans le cas de l'eau de 10 à 89°C, et en particulier de 25 à 70°C.
- la « percolation » ou « lixiviation » consiste à faire passer le solvant polaire à travers la tige d'inflorescence de poivrier, soit fraîche, soit préalablement séchée, et broyée.
- la distillation peut être effectuée par exemple par VMHD (hydrodistillation par micro-onde sous vide puisé). Lors de cette étape d'extraction, le solvant polaire est porté à une pression supérieure à la pression atmosphérique. En particulier lorsque le solvant est de l'eau, celui-ci peut être à l'état subcritique, c'est-à-dire que l'eau se trouve à une température comprise entre 100 et 374°C et à une pression comprise entre 1 et 218 bars. Dans ces conditions, l'eau se trouve sous forme liquide.

La concentration de l'extrait obtenu peut s'effectuer sous vide ou par évaporation d'une partie ou de la totalité du solvant ou en portant ledit solvant polaire à une température comprise dans une gamme allant de la température ambiante (25°C) à une température de 300°C au-dessus de la température d'ébullition du solvant utilisé, et de préférence à la température d'ébullition du solvant utilisé. Ainsi, le solvant polaire est au moins en partie réduit, voire totalement éliminé, notamment par évaporation, par sublimation, et de préférence sous pression réduite. On entend par « température d'ébullition du solvant », la température la plus élevée que le solvant peut atteindre avant de s'évaporer, sous forme gazeuse, librement. Cette température se calcule à la pression atmosphérique de 1 atmosphère (101,3 kPa).

L'étape d'extraction du procédé selon l'invention peut être continue ou discontinue, et peut comprendre une étape impliquant le chauffage, en particulier d'une durée allant de 1 seconde à 15 jours, et notamment de 10 secondes à 8 heures.

Lors d'une étape supplémentaire, l'extrait récupéré après extraction peut être stérilisé, notamment par chauffage avec ou sans pression et/ou par passage sur une membrane.

Cet extrait de tige d'inflorescence de poivrier peut également être désinfecté, notamment par chauffage avec ou sans pression ou encore par l'intermédiaire d'un agent désinfectant.

Enfin, cet extrait est avantageusement séché. Ce séchage peut être effectué par lyophilisation et/ou par atomisation.

Ce procédé d'extraction par solvant polaire permet ainsi d'obtenir un extrait comprenant au moins 1 % en poids, de préférence de 2 % à 15 % en poids, de préférence encore de 3 % à 10 %, de polyphénols totaux issus de la tige d'inflorescence (par rapport à l'extrait sec ou par rapport au poids de la tige d'inflorescence séchée).

La présente invention concerne l'utilisation de la composition à visée détoxifiante, ou du complément alimentaire ou aliment fortifié ou produit diététique tels que décrits ci-dessus, pour favoriser l'élimination par voie hépatique, des déchets de l'organisme.

Cette composition détoxifiante peut également être utilisée chez toute autre personne souhaitant détoxifier son organisme (lors d'un régime minceur, ou suite à un traitement médicamenteux, par exemple).

Les effets sont particulièrement surprenants, car :
- d'une part, la pipérine est habituellement extraite du fruit/graine du poivrier par solvant alcoolique ou CO₂ supercritique, or l'invention se rapporte à l'infusion, c'est-à-dire une extraction aqueuse des actifs de la tige de l'inflorescence de poivrier.
- d'autre part, car l'inflorescence du poivrier ne contient que très peu de pipérine (1,00 % en poids de matière sèche). Les résultats des analyses menées par l'Institut de Technologie Alimentaire (de Con Tho, Viet Nam) sont présentés dans le tableau 1 :

**Tableau 1**

| | Polyphénols | Pipérine |
|---|---|---|
| Baies de Poivre de Kampot (matières sèches) | 1,58 % | 4,01 % |
| Tige d'inflorescence de poivrier de Kampot (matières sèches) | 2,99 % | 1,00 % |

Pour ces analyses, les polyphénols et la pipéridine ont été extraits des baies ou tiges d'inflorescence de poivrier séchées, à l'éthanol à 96 % selon la méthode de la norme ISO 5564.

Les polyphénols ont été dosés par spectrophotométrie à 765 nm selon la méthode Folin-Ciocalteau Micro.

La pipérine a été dosée par spectrophotométrie à 343 nm selon la méthode de la norme ISO 5564.

Les résultats sont exprimés en % en poids de matière sèche.

Au vu de l'enseignement de la littérature, il ne pouvait donc pas être attendu que cette partie du poivrier, avec une teneur en pipérine quatre fois plus faible que celles des baies, puisse avoir un effet détoxifiant.

Pour ces deux raisons, la partie de plante utilisée et le procédé d'extraction, différents de ce qui est classiquement réalisé et retrouvé dans la littérature scientifique, l'effet détoxifiant de l'infusion de tige d'inflorescence de *Piper nigrum* n'était pas attendu.

L'invention a ainsi pour avantage de proposer une nouvelle composition ayant une action détoxifiante destinée à favoriser l'élimination des déchets de l'organisme, tout au moins aussi efficace que celles existantes sur le marché à l'heure actuelle.

La présente invention va être décrite plus en détail au moyen des exemples de réalisation ci-après destinés à illustrer l'invention sans n'aucunement en limiter la portée.

### EXEMPLES

Les tiges d'inflorescence de poivrier de Kampot mises en œuvre dans les exemples qui suivent ont subi :
- soit un séchage et un broyage pour obtenir des granulats grossiers utilisés notamment pour les préparations sous forme de "thé" ;
- soit un séchage, un broyage et un tamisage pour obtenir une poudre de granulométrie comprise entre 300 µm et 800 µm environ, et généralement proche de 500 µm ;
- soit une extraction par un solvant polaire pour obtenir un extrait liquide pouvant être réduit à sec.

### EXEMPLE 1

On prépare un thé de tige d'inflorescence de poivrier de Kampot Original séchée et broyée, selon le procédé d'infusion détaillé plus haut, et composé uniquement de tiges séchées et broyées d'inflorescence de poivrier de Kampot (2,5 g de matière sèche / 100 mL d'eau). Ce thé peut être utilisé comme détoxifiant hépatique en vue d'aider à éliminer les déchets de l'organisme.

### EXEMPLE 2

On prépare un thé composé de tige d'inflorescence de poivrier de Kampot séchée et broyée et de racine de Citronnelle reconnue pour ses propriétés toniques digestives (2,5 et 0,5 g de chaque matière sèche, respectivement, pour 100 ml d'eau). Ce thé est utilisé comme aide à la digestion, anti-flatulence et contre le ballonnement.

### EXEMPLE 3

On prépare un thé composé de tige d'inflorescence de poivrier de Kampot séchée et broyée et de racine de Gingembre (2,5 g de chaque matière sèche / 100 ml d'eau). Le gingembre est conseillé pour ses effets bénéfiques sur la circulation, et peut être utilisé comme tonique et aphrodisiaque.

### EXEMPLE 4

On prépare un thé composé de tige d'inflorescence de poivrier de Kampot séchée et broyée et de fleur de Jasmin (2,5 g de chaque matière sèche / 100 ml d'eau). Ce thé parfumé de Jasmin est utilisé comme relaxant, antistress et facilitant le sommeil.

### EXEMPLE 5

On prépare un thé composé de tige d'inflorescence de poivrier de Kampot séchée et broyée et de feuilles d'Orthosiphon (2,5 g de chaque matière sèche / 100 ml d'eau). Ce thé au goût minéral est utilisé comme aide à la minceur et éliminateur de l'acide urique.

### EXEMPLE 6

On prépare un complément alimentaire ou un aliment fortifié sous forme de poudre, conditionné ensuite en sachet (par exemple en stick), par mélange des ingrédients présentés dans le tableau 2 :

**Tableau 2**

| Ingrédients | Par stick en mg |
|---|---|
| Poudre de tige d'inflorescence de poivrier | 500,00 |
| Fructo oligosaccharides | 1200,00 |
| (Arôme) | (10,00) |
| Edulcorant | 5,00 |

Ledit complément alimentaire ou aliment fortifié est apte à être dilué dans un verre d'eau ou bien peut être ingéré directement par l'Homme.

### EXEMPLE 7

Le complément alimentaire ou l'aliment fortifié renfermant les ingrédients présentés dans le tableau 3 est conditionné dans un bouchon apte à être vissé sur une bouteille d'eau de 300 mL, 1 L ou 1,5 L.

**Tableau 3**

| Ingrédients | Par pot en mg |
|---|---|
| Poudre de tige d'inflorescence de poivrier | 700,00 |
| Inuline | 1820,00 |
| (Arôme) | (25,00) |
| Edulcorant | 5,00 |
| Colorant | 10,00 |
| Maltodextrine | qsp |

Afin d'utiliser ce complément ou cet aliment fortifié, il suffit à l'utilisateur de visser le bouchon contenant ladite composition sur une bouteille de 300 mL, 1 L ou 1,5 L contenant par exemple de l'eau. La dose recommandée pour l'individu sera ensuite de 1 bouchon par jour.

### EXEMPLE 8

Après mélange des ingrédients du tableau 4 et placement dans une ampoule, on obtient, après traitement à l'autoclave et selon des techniques connues de l'homme de métier, un complexe composé d'un extrait alcoolique éthanol/eau de tige d'inflorescence de poivrier séchée (2,5 g dans 100 mL d'un mélange éthanol/eau à 70/30 en volume) et d'un extrait alcoolique éthanol/eau de feuilles d'orthosiphon séchées (1 g dans 100 mL d'un mélange éthanol/eau à 70/30 en volume). Les extraits sont filtrés avant mise en ampoule.

**Tableau 4**

| Ingrédients | Par ampoule |
|---|---|
| Extrait de tige d'inflorescence de poivrier | 2 mL |
| Extrait de feuilles d'orthosiphon | 1 mL |
| Eau | 5 mL |

La dose d'administration recommandée est d'une ampoule par jour à diluer dans un verre d'eau.

### EXEMPLE 9

Après mélange des ingrédients présentés dans le tableau 5 et extrusion du mélange, selon des techniques connues de l'homme du métier, un complément alimentaire ou un aliment fortifié sous forme de granulés extrudés est obtenu.

**Tableau 5**

| Ingrédients | Par pot (en mg) |
|---|---|
| Poudre de tige d'inflorescence de poivrier | 18000,00 |
| Poudre de gingembre | 14000,00 |
| Inuline | 128 000,00 |

La dose journalière recommandée est de 2,6 g de granulés, dosés par exemple avec une cuillère à café et à diluer dans un verre d'eau.

### EXEMPLE 10

Des gélules sont préparées selon la formulation présentée dans le tableau 6.

**Tableau 6**

| Ingrédients | Par gélule en mg |
|---|---|
| Poudre de tige d'inflorescence de poivrier | 95,00 |
| Poudre de citronnelle | 65,00 |
| Excipients : | |
| Silice colloïdale QS | 1,00 |
| Stéarate de magnésium QS | 1,00 |

La dose journalière recommandée est de 1 gélule par jour.

### EXEMPLE 11

Des comprimés sont préparés selon la formulation présentée dans le tableau 7 ci-dessous.

**Tableau 7**

| Ingrédients | Par comprimé en mg |
|---|---|
| Poudre de tige d'inflorescence de poivrier | 400,00 |
| Poudre de jasmin | 300,00 |

| Excipients : | |
|---|---|
| Cellulose microcristalline | 95,00 |
| Silice colloïdale | 5,00 |
| Stéarate de magnésium | 14,00 |

La dose journalière recommandée est de 1 comprimé par jour.

### EXEMPLE 12

Un extrait aqueux de tige d'inflorescence de poivrier est réalisé par percolation de tiges séchées (2,5 g pour 100 mL d'eau) puis l'extrait est évaporé jusqu'à siccité.

Un extrait aqueux de feuilles de d'orthosiphon (1 g pour 100 mL d'eau) est réalisé par percolation de feuilles séchées puis l'extrait est évaporé jusqu'à siccité.

**Tableau 8**

| Ingrédients | Par flacon en mg |
|---|---|
| Extrait sec de tige d'inflorescence de poivrier | 7500,00 |
| Extrait sec de feuilles d'orthosiphon | 4000,00 |
| (Arôme) | (4000,00) |
| Edulcorant | 500,00 |
| Acide citrique pH<4,5 | 500,00 |
| Sorbate de potassium | 500,00 |
| Benzoate de sodium | 500,00 |

Le complément alimentaire, le produit diététique ou l'aliment fortifié selon cet exemple est, après mélange homogène des ingrédients du tableau 8, conditionné dans un flacon avec gobelet doseur. La dose journalière recommandée est de 25 mL (à doser grâce au gobelet doseur) et à diluer dans un verre d'eau.

### EXEMPLE 13 : test chez la souris

L'effet d'une infusion de poudre de tige d'inflorescence de poivre de Kampot a été évalué chez des souris Swiss albino. Les animaux ont reçu l'infusion (2,5 g de matière sèche / 100 mL d'eau) incorporée dans leur eau de boisson, à des doses de 10 % et 20 % en volume, pendant 20 jours. Les résultats obtenus mettent en évidence une augmentation significative et dose-dépendante dans l'organisme du taux de gluthathion S-transférase (GST) et d'acide soufré (-SH). La GST catalyse la conjugaison du glutathion sur les composés électrophiles mutagéniques et carcinogéniques. De plus, la GST est capable de se lier à une grande variété de toxiques. Les résultats obtenus ont montré que des niveaux élevés de cytochromes b5 et P450, enzymes de phase I, étaient également statistiquement significatifs et dose-dépendants. Le taux de malondialdéhyde (MDA), marqueur de la peroxydation lipidique, était plus faible dans le groupe 20 % d'infusion. L'ensemble de ces résultats suggère donc un effet de la composition selon l'invention sous forme d'infusion sur l'induction du système de détoxification des enzymes de phase I et de phase II.

Bien que l'invention ait été décrite en relation avec un mode de réalisation particulier, il est bien évident qu'elle n'y est nullement limitée et qu'elle comprend tous les équivalents techniques des moyens décrits ainsi que leurs combinaisons si celles-ci entrent dans le cadre des revendications ci-après.

## Revendications

1. Composition à visée détoxifiante pour son utilisation par administration par voie orale en tant que détoxifiant en vue de favoriser l'élimination par voie hépatique des déchets de l'organisme, **caractérisée en ce qu'**elle comprend des tiges d'inflorescence de poivrier séchées, éventuellement broyées, et éventuellement tamisées et/ou un extrait, aqueux ou sec, de tiges d'inflorescence de poivrier.

2. Composition pour son utilisation selon la revendication 1dans laquelle les tiges d'inflorescence de poivrier sont choisies parmi les tiges d'inflorescence de poivrier *Piper nigrum* ou *Piper longum,* plus particulièrement les tiges d'inflorescence de poivrier de la région de Kampot, au Cambodge.

3. Composition pour son utilisation selon l'une quelconque des revendications précédentes, dans laquelle ladite composition renferme au moins 1 % en poids, de préférence de 2 % à 15 % en poids, de préférence encore de 3 % à 10 %, de polyphénols totaux issus de la tige d'inflorescence.

4. Composition pour son utilisation selon l'une quelconque des revendications précédentes, dans laquelle ladite composition se présente sous forme buvable, telle que sous la forme d'une infusion, d'une décoction, d'une macération, d'une boisson aromatisée ou toute autre forme buvable.

5. Complément alimentaire ou aliment fortifié ou produit diététique comprenant la composition selon l'une quelconque des revendications précédentes dans des proportions pondérales comprises entre 0,1 % et 85 %, de préférence entre 3 % et 65 %, de préférence encore entre 20 % et 60 % pour son utilisation par administration par voie orale en tant que détoxifiant en vue de favoriser l'élimination par voie hépatique des déchets de l'organisme,

6. Complément alimentaire ou aliment fortifié ou produit diététique pour son utilisation selon la revendication 5, **caractérisé en ce qu'**il comprend en outre au moins un actif choisi parmi : les vitamines, telle que les vitamines B₆ ou C ; les caroténoïdes, tels que le bêta-carotène ou la lutéine ; les oligoéléments minéraux, tels que le magnésium, le calcium ou le fer ; ou des extraits de romarin, de chardon marie, de radis noir, de la reine des prés, de l'ortie, de la prêle, des feuilles d'orthosiphon, de la citronnelle, du gingembre, du jasmin; des polyphénols et/ou des anthocyanes additionnels.

7. Complément alimentaire ou aliment fortifié ou produit diététique pour son utilisation selon l'une des revendications 5 ou 6, **caractérisé en ce qu'**il se présente sous forme de comprimés nus ou enrobés, de comprimés multicouches, de comprimés effervescents, de dragées, de gélules, de capsules, de poudre, de granules, de sticks, de pastilles, de capsules molles, d'une solution ou d'une suspension aqueuse dans une ampoule en verre, en stick ou en boisson, en flacon compte-gouttes ou en spray.

8. Procédé de préparation de la composition selon l'une quelconque des revendications 1 à 4, **caractérisé en ce qu'**il comprend au moins l'une des étapes i) ou ii) suivantes de :
i) récolte, séchage, éventuel broyage et éventuel tamisage, de tiges d'inflorescence de poivrier ;
ii) extraction de tiges d'inflorescence de poivrier fraîches et/ou séchées au moyen d'un solvant aqueux, puis filtration de l'extrait, en option concentration de l'extrait, éventuellement jusqu'à siccité.

## Patentansprüche

1. Entgiftungszusammensetzung für ihre Verwendung zur oralen Verabreichung als Entgiftungsmittel zwecks Förderung der Ableitung von Abfallstoffen aus dem Organismus über den Weg der Leber, **dadurch gekennzeichnet, dass** sie getrocknete, eventuell zerkleinerte und eventuell gesiebte Pfefferstrauch-Blütenstandsstängel und/oder einen wässrigen oder trockenen Extrakt von Pfefferstrauch-Blütenstandsstängeln umfasst.

2. Zusammensetzung für ihre Verwendung nach Anspruch 1, wobei die Pfefferstrauch-Blütenstandsstängel aus den Pfefferstrauch-Blütenstandsstängeln *Piper nigrum* oder *Piper longum,* genauer den Pfefferstrauch-Blütenstandsstängeln aus der Region Kampot in Kambodscha, ausgewählt sind.

3. Zusammensetzung für ihre Verwendung nach einem der vorangehenden Ansprüche, **dadurch gekennzeichnet, dass** die Zusammensetzung mindestens 1 Gew.-%, vorzugsweise 2 bis 15 Gew.-%, noch vorzugsweiser 3 bis 10 % Polyphenol insgesamt aus dem Blütenstandsstängel einschließt.

4. Zusammensetzung für ihre Verwendung nach einem der vorangehenden Ansprüche, **dadurch gekennzeichnet, dass** sie in trinkbarer Form wie in Form einer Infusion, einer Abkochung, einer Mazeration, eines aromatisierten Getränks oder jeder anderen trinkbaren Form vorliegt.

5. Nahrungsergänzung oder angereichertes Lebensmittel oder Diätprodukt, umfassend die Zusammensetzung nach einem der vorangehenden Ansprüche in Gewichtsanteilen zwischen 0,1 % und 85 %, vorzugsweise zwischen 3 % und 65 %, noch vorzugsweiser zwischen 20 % und 60 % für ihre Verwendung zur oralen Verabreichung als Entgiftungsmittel zwecks Förderung der Ableitung von Abfallstoffen aus dem Organismus über den Weg der Leber.

6. Nahrungsergänzung oder angereichertes Lebensmittel oder Diätprodukt für seine Verwendung nach Anspruch 5, **dadurch gekennzeichnet, dass** sie/es ferner mindestens einen Wirkstoff umfasst, der ausgewählt ist aus: den Vitaminen wie den Vitaminen B₆ oder C; den Karotinoiden wie dem Beta-Karotin oder dem Lutein; den mineralischen Spurenelementen wie dem Magnesium, dem Kalzium oder dem Eisen; oder den Extrakten aus Rosmarin, von Mariendistel, Schwarzem Rettich, Mädesüß, Brennnessel, Schachtelhalm, Orthosiphonblättern, Zitronengras, Ingwer, Jasmin; der Polyphenole und/oder der Anthocyane als Zugabe.

7. Nahrungsergänzung oder angereichertes Lebensmittel oder Diätprodukt für ihre/seine Verwendung nach einem der Ansprüche 5 oder 6, **dadurch gekennzeichnet, dass** sie/es in Form bloßer oder umhüllter Tabletten, mehrschichtiger Tabletten, Sprudeltabletten, Dragees, Gelkapseln, Kapseln, Pulver, Granulat, Sticks, Pastillen, weichen Kapseln, einer Lösung oder wässrigen Suspension in einer Glasampulle, Stick oder Getränk, im Tropfenzählerflakon oder als Spray vorliegt.

8. Verfahren zur Herstellung der Zusammensetzung nach einem der Ansprüche 1 bis 4, **dadurch gekennzeichnet, dass** es mindestens einen der folgenden Schritte i) oder ii) umfasst:
i) Ernten, Trocknen, eventuell Zerkleinern und eventuell Sieben von Pfefferstrauch-Blütenstandsstängeln;
ii) Extrahieren von frischen und/oder getrockneten Pfefferstrauch-Blütenstandsstängeln mittels eines wässrigen Lösungsmittels, dann Filtrieren des Extrakts, optional Konzentrieren des Extrakts, eventuell bis zur Trockenheit.

## Claims

1. Detoxifying composition for use thereof via oral administration route as detoxifier with a view to promoting elimination of body waste via hepatic route, **characterized in that** it comprises dried pepper plant inflorescence stems, optionally ground, and optionally screened and/or an aqueous or dry extract of pepper plant inflorescence stems.

2. Composition for use thereof according to claim 1, wherein the pepper plant inflorescence stems are selected from among the inflorescence stems of the pepper plant *Piper nigrum* or *Piper longum,* more particularly the pepper plant inflorescence stems from the Kampot region in Cambodia.

3. Composition for use thereof according to any of the preceding claims, wherein said composition contains at least 1 weight %, preferably 2 to 15 weight %, more preferably 3 to 10 weight % of total polyphenols derived from the inflorescence stem.

4. Composition for use thereof according to any of the preceding claims, wherein said composition is in drinkable form, such as in the form of an infusion, decoction, maceration, flavoured beverage or any other drinkable form.

5. Food supplement or fortified food or dietary product comprising the composition according to any of the preceding claims in weight proportions of between 0.1 % and 85 %, preferably between 3 % and 65 %, more preferably between 20 % and 60 % for use thereof via oral administration route as detoxifier with a view to promoting the elimination of body waste via hepatic route.

6. Food supplement or fortified food or dietary product for use thereof according to claim 5, **characterized in that** it further comprises at least one active substance selected from among: vitamins such as vitamins B₆ or C; carotenoids such as beta-carotene or lutein; mineral trace elements such as magnesium, calcium or iron; or extracts of rosemary, milk thistle, black radish, meadowsweet, nettle, horsetail, orthosiphon leaves, lemon grass, ginger, jasmine; additional polyphenols and/or anthocyans.

7. Food supplement or fortified food or dietary product for use thereof according to one of claims 5 or 6, **characterized in that** it is in the form of coated or uncoated tablets, multilayer tablets, effervescent tables, sugar-coated tablets, capsules, hard capsules, powder, granules, sticks, lozenges, soft capsules, an aqueous solution or suspension in a glass vial, stick or beverage, dropper bottle or spray.

8. Method for preparing the composition according to any of claims 1 to 4, **characterized in that** it comprises at least one of following steps i) or ii):
i) harvesting, drying, optional grinding and optional screening of pepper plant inflorescence stems;
ii) extracting fresh and/or dried pepper plant inflorescence stems by means of an aqueous solvent, followed by filtering the extract, optionally concentrating the extract optionally to dryness.
